# EUROPEAN PATENT SPECIFICATION

(11) **EP 0 642 343 B1**
(45) Date of publication and mention of the grant of the patent: **30.08.2000**
(21) Application number: 93913256.9
(22) Date of filing: 28.05.1993
(51) Int. Cl.: A61K 31/435

(54) **Acridine carboxamides for the treatment of cancer**
ACRIDIN CARBOXAMIDE ZUR BEHANDLUNG VON KREBS
Acridine carboxamides pour le traitement des cancers

(30) Priority: 28.05.1992 NZ 24293892; 22.01.1993 US 7690
(43) Date of publication of application: 15.03.1995
(73) Proprietor: XENOVA LIMITED, Slough, Berkshire SL1 4EF (GB)
(72) Inventor: BAGULEY, Bruce, Charles 36a Olsen Avenue, Auckland 1004 (NZ); ATWELL, Graham, John 192 Gowing Drive, Auckland 1005 (NZ); DENNY, William, Alexander 165 Gossamer Drive, Auckland 1706 (NZ); FINLAY, Graeme, John 58 Glenvar Road, Auckland 1310 (NZ); REWCASTLE, Gordon, William 107 Grande Vue Road, Auckland 1702 (NZ)
(74) Representative: Woods, Geoffrey Corlett
(86) International application number: GB9301150
(87) International publication number: WO9324096

(56) References cited:
- EP-A- 0 098 098
- EP-A- 0 172 744
- EP-A- 0 206 802
- CHEM. NEW ZEAL. vol. 53, no. 6, 1989, pages 145 - 150 G.W. REWCASTLE ET AL 'Synthesis and development of two new classes of anticancer drugs: the tricyclic carboxamides and the xanthenoneacetic acids.'
- EUR. J. CANCER CLIN. ONCOL. vol. 24, no. 11, 1988, pages 1783 - 1790 E. SCHNEIDER ET AL 'Cell line selectivity and DNA breakage properties of the antitumour agent N-Ä2-(dimethylamino)ethyl Üacridine-4-carboxamide: role of DNA Topoisomerase II.'
- J. NATL. CANCER INST. vol. 82, no. 5, 1990, pages 398 - 402 B.C. BAGULEY ET AL 'Design of DNA intercalators to overcome topoisomerase II-mediated multidrug resistance.'
- CANCER RES. vol. 47, no. 2, 1987, pages 424 - 432 F. TRAGANOS ET AL 'Effects of a new amsacrine derivative, N-5-dimethyl-9-(2-me thoxy-4-methylsulfonylamino)phenylamino-4 acridinecarboxamide, on cultured mammalian cells.'
- Baguley, B.C., et al., Cancer Chemother. and Pharmacol., 36, 1995, 244-48

## Description

The present invention relates to the treatment of tumours, especially to the treatment of melanoma and cancer of the colon, and to the circumvention of multidrug resistance in cancer treatments.

For certain types of cancer, chemotherapy has been capable of rendering patients with responsive tumours free of disease. However, this responsive category does not include the most frequently encountered forms of malignant tumours.

The most common types of cancer in western populations are colon, lung and breast cancer. Each of these can be treated to some extent with existing chemotherapy, with different drugs being used preferentially for each type of malignancy (for instance, doxorubicin, cyclophosphamide and methotrexate for breast cancer, 5-fluorouracil for colon cancer), but response rates are not good. In addition, melanoma is a disease which is increasing in incidence at an alarming rate among fair-skinned populations. In melanoma, only 25-30% of patients with disseminated disease respond to treatment, and only 5-10% sustain durable remission (Evans B.D., et al., Proc. Am. Soc. din. Oncol. 1990, **9**, 276).

There is therefore a great need for new types of cancer therapy, and a desperate need for such treatments for the above cancers in particular.

The basis for the development of the majority of anticancer drugs used today has been a panel of mouse tumours including transplantable leukaemias, the Lewis lung carcinoma and the colon 38 adenocarcinoma. A number of human tumour xenografts in mice have also been used. In general, the leukaemias are the most sensitive to experimental agents, the xenografts are the most resistant and the Lewis lung and colon 38 are of intermediate sensitivity (Goldin A., et al., Eur. J. Cancer 1981, **17**, 129-142).

The murine Lewis lung adenocarcinoma is a tumour which initially arose spontaneously in C₅₇Bl mice and which has a number of features which make it a good model for clinical carcinomas. It grows easily both in vitro and in vivo, and is aneuploid, heterogeneous, metastatic and resistant to many but not all clinical antitumour agents. Zacharski (Zacharski L.R., Haemostasis 1986, **16**, 300-320) has concluded that although Lewis lung has the cytological appearance of a large-cell cancer, its rapid rate of growth, propensity to cause lethal metastases, as well as its susceptibility to combination chemotherapy, radiation and anticoagulant treatment, make it a good model for human small-cell lung cancer (SCLC). The colon 38 tumour arose in carcinogen-treated mice, and because it is sensitive to 5-fluorouracil it can be considered as a useful model for human colon cancer (Corbett T.H., et al., Cancer Chemother. Rep. 1975, **5**, 169-186, and Cancer 1977, **40**, 2660-2680). Human melanoma xenografts have been considered for some time as an appropriate model for the development of new anticancer drugs for melanoma (Taetle R., et al., Cancer 1987, **60**, 1836-1841).

The essence of treating cancer with cytotoxic anticancer drugs is to combine a mechanism of cyto-toxicity with a mechanism of selectivity for tumour cells over host cells. The selectivity of a drug for a particular cancer will depend on the expression by that cancer of properties which promote drug action, and which differ from tumour to tumour.

Most of the currently available cytotoxic drugs can be broadly divided into four groups: those which react chemically with DNA (such as the alkylating agents and cisplatin and bleomycin), those which disrupt DNA synthesis (such as the antimetabolites), those which disrupt the mitotic apparatus (such as the vinca alkaloids and taxol) and those which are directed against the cellular enzyme topoisomerase II ("topo II") in order to effect changes in the topological form of the DNA.

DNA topoisomerases were named after the first method used to detect their activity. When incubated with closed circles of double-stranded DNA prepared from viruses or bacteria, topoisomerases enzymatically change the number of coils contained in each circle (circular forms of DNA with different degrees of coiling are called topo-isomers). The topoisomerases are perhaps better understood as enzymes which temporarily break one strand of the DNA double helix (topoisomerase I or "topo I") or which simultaneously break two strands of the DNA double helix ("topo II") in order to effect changes in the topological form of the DNA.

Topoisomerases have two main functions in the cell. The first is to act as swivel points on the DNA in association with DNA and RNA polymerases during the biosynthesis of nucleic acids required for cell replication and gene expression. The second is to untangle the DNA strands of the daughter chromosomes following DNA replication prior to cell division. The DNA of chromosomes is organised as a series of loops on a protein-aceous "scaffold". After duplication of chromosomes and of the "scaffolds", the DNA loops must be separated. Since there are hundreds of thousands of DNA loops attached to each chromosome scaffold it is not hard to imagine the necessity for an enzyme which effectively removes tangles by passing one double DNA strand through another. This process absolutely requires topo II.

Topo I acts by transiently breaking a DNA strand and attaching itself to one of the free ends of the broken DNA via the amino acid tyrosine. Topo II contains two identical protein subunits, each of which is capable of breaking a DNA strand and attaching itself to one of the free ends. With both DNA strands broken, a second DNA double helix can be allowed to pass between the two enzyme protein subunits, thus allowing not only swivelling but also untangling of DNA. The process is normally spontaneously reversible by cleavage of the enzyme-DNA links and re-sealing of DNA breaks to restore the DNA to its original form.

Topo II-directed agents include a number of important clinical anti-cancer drugs such as anthracycline antibiotics (e.g. doxorubicin), epipodophyllotoxin derivatives (e.g. etoposide) and synthetic DNA intercalating drugs (e.g. amsacrine). These act by jamming the enzyme in its DNA-associated form (Liu L.F., Annu. Rev. Biochem. 1989, **58**, 351-375). Such molecular lesions might be expected to be innocuous, since the drug eventually dissociates itself from the complex and the DNA strand breaks are then repaired perfectly. However, in a small proportion of cases, the presence of drug causes the complex to be dissociated abnormally, generating some kind of DNA lesion which eventually leads to cell death.

Although the antitumour activity of many of these agents has been known for many years, it is only since 1984 that the molecular target of action has been identified (Nelson E.M., Tewey K.M., Liu L.F., Proc. Natl. Acad. Sci. USA 1984, **81**, 1361-1364 and Tewey K.M., et al., Science 1984, **226**, 466-468).

A number of mechanisms of resistance to topo II poisons have now been identified, and in many cases the development of resistance to one drug is accompanied by the simultaneous acquisition of resistance to a variety of other drugs. Since the mechanism of resistance determines the pattern of cross-resistance to other drugs, an understanding of these processes is of great importance to the strategy for the use of these agents. Several resistance mechanisms important to the use of these agents have now been characterised in experimental systems, including those involved in drug transport (Endicott J.A., Ling V., Annu. Rev. Biochem 1989, **58**, 351-375), drug-target interaction (Beck W.T., Biochem, Pharmacol. 1987, **36**, 2879-2888) and drug detoxification (Deffie A.M., et al., Cancer Res. 1988, **48**, 3595-3602).

Attempts to overcome multidrug resistance (mdr) clinically have been concerned mainly with the first of these mechanisms, a drug transport mechanism that pumps drug out of cells. Various inhibitors of this process, such as verapamil, are known and some have been used in combination with drugs such as doxorubicin and etoposide to treat cancer (Stewart D.J., Evans W.K., Cancer Treat. Rev. 1989, **16**, 1-10, Judeon I.R., Eur. J. Cancer 1992, **28**, 285-289).

Another approach is to design drugs which can overcome mdr. We have now discovered that the investigational drug acridine carboxamide ("DACA") appears to be one such drug.

The compound tested was the dihydrochloride of N-[2-(dimethylamino)ethyl]acridine-4-carboxamide of formula 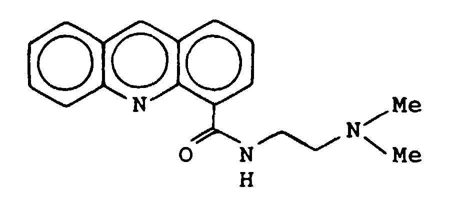 and is described and claimed in EP 98098 (USP 4590277). That patent also describes and claims other acridine carboxamide compounds and their use for the treatment of tumours; more particularly, the treatment of Lewis lung tumours and leukaemia is described.

Various other derivatives of DACA have been tested for their antitumour activity and the results are reported in the literature. Active compounds are carboxamides having an unsubstituted or substituted aromatic ring system comprising two or more fused rings and having an oxygen or an aromatic nitrogen peri to the carboxamide side chain. As well as the other acridine carboxamides (EP 98098/USP 4590277, Denny W.A. *et al*., J. Med. Chem. 1987_{;} **30**: 658-663 and Gordon W Rewcastle *et al*, Chemistry in New Zealand, vol. 53, No. 6, December 1989), examples are phenyl quinoline and pyrido quinoline carboxamides (EP 206802 A/USP 4904659, Atwell G.J. *et al*., J. Med. Chem. 1988; **31**: 1048-1052 and Atwell G.J. *et al*., J. Med. Chem. 1989; **32**: 396-401), phenazine carboxarnides (EP 172744 A/US Application 765993 filed 1985 and Rewcastle G.W. *et al*., J. Med. Chem. 1987; **30**: 843-851), carboxamides having angular tricyclic chromophores: phenanthridine carboxamides (NZ Patent 215286, 1986 and Atwell G.J., et al., J. Med. Chem. 1988; **31**: 774-779) and carboxamides having various linear tricyclic chromo-phores, including dibenzo[1,4]dioxin carboxamides (Palmer B.D., et al., J. Med. Chem. 1988; **31**: 707-712, Lee et al., J. Med. Chem. 1992; **35**, 258-266, Palmer et al, J. Org. Chem. 1990; **55**: 438-441, Lee et al., J. Chem. Soc., Perkin Trans. 1, **1990**, 1071-1074, and Cambie et al., J. Organomet. Chem., 1991: **420**, 387 ff.). These other compounds are structurally very similar to DACA and are able to act in the same way.

DACA is a DNA-binding drug which acts at the same target, topoisomerase II, as do drugs such as amsacrine and etoposide. We have now found, however, that it has a different in vitro cytotoxicity profile to these compounds and a number of advantages over existing clinical drugs in the class of topoisomerase-directed agents.

Firstly, it is active against cell lines displaying both P-glycoprotein-mediated or "transport" resistance and "atypical" or "altered" multidrug resistance. In this respect it is unique among topo II inhitibors. For instance,Schneider *et al* report in Eur. J. Cancer Clin. Oncol., vol 24 no. 11, 1988, 1783-1790 that, in *in vitro* tests, DACA shows no cross-resistance to P/ACTD cells which are cross-resistant to vincristine and have the characteristic of a multi-drug resistance line.

DACA and related compounds may therefore be used to circumvent mdr. For this it may be used in combination with other cytotoxic drugs, more especially those that kill cells by a different cytotoxic mechanism, and/or as a second-line treatment if first-line treatment fails because of the development of multidrug resistance.

Secondly, we have found that, unexpectedly, DACA is effective against advanced colon 38 tumours and an advanced melanoma xenograft in mice colon when administered in a divided dose schedule over a period of two hours. (In this context "advanced tumour" means that the tumour was more than 5 mm in diameter at the time of measurement.) In contrast, a single administration of DACA at the maximum tolerated dose (150 mg/kg), which is curative against Lewis lung tumours growing as lung nodules in mice, was only marginally effective.

Thirdly, DACA has the ability to cross the blood brain barrier, suggesting that rapidly growing brain tumours may also be treatable, more particularly when administered in a divided dose schedule.

Accordingly the present invention provides the use of a tumour-inhibiting compound which is an acridine carboxamide of the formula (I') 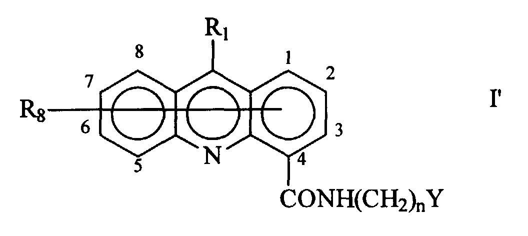
wherein R₁ is H, CH₃ or NHR₀, where Rₒ is H, COCH₃, SO₂CH₃, COPh, SO₂Ph or C₁-C₅ alkyl which is unsubstituted or substituted by hydroxy, C₁-C₅ alkoxy or amino;
n is 2;
R₈ is H or is one or two substituents selected from CH₃, OCH₃, halogen, CF₃, NO₂, NH₂, NHCOCH₃, and NHCOOCH₃ at positions 1-3 and 5-8; and
Y is C(NH)NH₂, NHC(NH)NH₂, or NR₄R₅, wherein each of R₄ and R₅ is H or C₁-C₅ alkyl which is unsubstituted or substituted by hydroxyl or amino; or a physiologically tolerable acid addition salt or N-oxide thereof; in the manufacture of a medicament for the treatment of tumours by a divided-dose schedule comprising a first administration and a second administration of the sad compound, wherein the second administration commences between 15 minutes and 24 hours after commencement of the first administration.

The invention also provides the use of a tumour-inhibiting compound which is an acridine carboxamide of the formula (I') 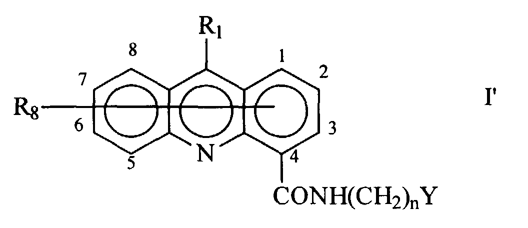
wherein R₁ is H, CH₃ or NHR₀,
where Rₒ is H, COCH₃, SO₂CH₃, COPh, SO₂Ph or C₁-C₅ alkyl which is unsubstituted or substituted by hydroxy, C₁-C₅ alkoxy or amino;
n is 2;
R₈ is H or is one or two substituents selected from CH₃, OCH₃ halogen, CF₃, NO₂, NH₂, NHCOCH₃, and NHCOOCH₃ at positions 1-3 and 5-8; and
Y is C(NH)NH₂, NHC(NH)NH₂, or NR₄R₅, wherein each of R₄ and R₅ is H or C₁-C₅ alkyl which is unsubstituted or substituted by hydroxyl or amino; or a physiologically tolerable acid addition salt or N-oxide thereof; in the manufacture of a medicament for circumventing multidrug resistance in the treatment of tumours by a divided dose schedule comprising a first administration and a second administration of the said compound wherein the second administration commences between 15 minutes and 24 hours after commencement of the first administration.

There may, for example, be at least 2 administrations in total in the divided dose, administrations being preferably at least every 2 hours, for example every hour or every ½ hour, for up to 4 hours or longer.

In one preferred aspect of the invention the divided dose schedule comprises at least 2 administrations of drug over a period of from 30 minutes to 8 hours. In another aspect the schedule comprises at least 2 administrations of drug over a period of from 30 minutes to 6 hours, or from 30 minutes to 4 hours. More preferably the schedule comprises 2 to 4 administrations of drug over a period of up to 4 hours, for example 2 to 4 hours.

We believe also that suitable DNA-binding compounds will reduce the toxicity of DACA when administered in conjunction with a divided high-dose schedule of DACA. DNA-binding compounds include, for example 9-amino-acridine; such compounds have the ability to inhibit the antitumour activity of DACA.

Thus in one aspect of the invention at least one of the constituent doses of the acridine carboxamide of formula (I') or salt or N-oxide thereof is used with a further component which is a DNA-binding agent that reduces the host toxicity of the first component, this further component and the acridine carboxamide of formula I' or salt or N-oxide thereof being present together in the same formulation or in separate formulations for simultaneous or sequential administration.

Doses of DACA in mice of 100 to 300 mg/kg, especially 150 to 250 mg/kg, more especially substantially 200 mg/kg, administered as a divided dose over a period of 2 to 4 hours, have proved suitable. Administration to humans of, for example, substantially 800 mg/m² of DACA or equivalent amount of other carboxamide should be mentioned, but lower or higher amounts may also be possible. As explained above, advantageous results are obtained when the dose is administered as a divided dose, producing a high plasma level followed by a drop in level and then a high level again. Thus, the use of substantially 800 mg/m² for the total of the constituent doses of a divided dose should be mentioned.

The compounds used in the invention may be prepared by methods known *per se*, for example by methods described in the relevant literature references mentioned above and in EP 98098 A (US 4590277), in EP 206802 A (US 4904659) and in EP 172744 A (US Application 765993 filed 1985) or by analogous methods.

When used herein, the term "C₁-C₅ alkyl" denotes an alkyl group having from 1 to 5, preferably 1 to 4, carbon atoms.

An amino function as substituent of a C₁-C₅ alkyl radical represented by Rₒ may be unsubstituted or, for example, substituted by one or two C₁-C₅ alkyl groups (where C₁-C₅ alkyl has the meaning given above), especially by one or two methyl groups. Thus, for example, an amino substituent of a C₁-C₅ alkyl radical represented by Rₒ may be NH₂, NHCH₃ or N(CH₃)₂.

A C₁-C₅ alkoxy group as substituent of a C₁-C₅ alkyl radical represented by Rₒ is especially a methoxy group.

Examples of substituted C₁-C₅ alkyl groups are those substituted by hydroxy, C₁-C₅ alkoxy or an amino function, for example C₁-C₅ alkyl optionally substituted with hydroxy, amino, methylamino, dimethylamino or methoxy.

A preferred subclass of compounds of formula I' are those where
- R₁: represents H or NH₂,
- R₈: represents up to two of 1-, 5-, 6-, 7-, and 8-NO₂, 5- and 6-CH₃ and 5-Cl,
- Y: represents NHC(NH)NH₂, N(CH₃)₂, or NHCH₂CH₂OH, and
- n: represents 2.

Compounds specifically identified in EP 98098 A, EP 206802 A and EP 172744 A and in the literature references given above should also be mentioned, including not only DACA, but also N-[2-(dimethylamino)ethyl]-9-raethoxyphenazine-1-carboximide, N-[2- (dimethylamino)-ethyl]-2-(4-pyridyl)quinoline-8-carboxamide, N-[2-(dimethylamino}ethyl]-2-phenylquinoline-8-carboxamide and N-[2-(dimethylamino)ethyl]-9-bromo-dibenzo[1,4]dioxin-1-carboxamide. The latter compound and other substituted dibenzo[1,4]dioxin-1-carboxamides are disclosed in J. Med. Chem. 1992, 35, 258-266.

The compounds used according to the invention form pharmaceutically acceptable addition salts with both organic and inorganic acids. Examples of suitable acids for salt formation are hydrochloric, sulphuric, phosphoric, acetic, citric, oxalic, malonic, salicylic, malic, fumaric, succinic, ascorbic, maleic and methanesulphonic acids.

The administration of an acridine carboxamide of the general formula I, or salt or N-oxide thereof, may include the administration of a second tumour-inhibiting compound that is not a topo-II inhibitor. Thus, an acridine carboxamide such as DACA could be given, for example, up to ± 2 days of a second drug, or alternatively could be given as a separate or alternating course with another cytotoxic drug and separated by a period of bone marrow or other host tissue recovery, generally 3 to 4 weeks. DACA may, for example, be administered by intravenous infusion using the divided dose regime mentioned above, for example as a series of 2 to 4 administrations over a period of 2 to 4 hours.

Suitable tumour inhibiting compounds that are not topo-II inhibitors are, for example, cisplatin, cyclophosphamide, bleomycin, carboplatin, decarbazine, mitomycin C and melphalan. Suitable DNA synthesis inhibitors are, for example, 5-fluorouracil, 5-fluorodeoxyuridine and methotrexate; and suitable agents that disrupt the mitotic apparatus are, for example, taxol and suitable Vinca alkaloids, for example, vincris-tine, vinblastine and vindesine. Other non-topo II inhibitors that may be used include, for example, tamoxifen (which acts by preventing the action/synthesis of hormones), tissue necrosis factor, Interleukin II and Interferon. These agents should be used in a treatment schedule which has been found optimal for antitumour effect; for example, cyclophosphamide may be used at monthly intervals, and vincristine at monthly or weekly intervals.

There are four main types of multidrug resistance related to topo II inhibitor:
(a) No change in the topoisomerase II enzyme but increased transport of the drug out of the cell. DACA is not susceptible to this, while etoposide and doxorubicin are.
(b) No change in the topoisomerase II enzyme, but an increase in drug detoxifying enzymes. This applies to doxorubicin but not to etoposide or DACA.
(c) A guantitative change (decrease) in the amount of topoisomerase II enzymes. Since the amount of topo II is regulated during the cell division cycle, cytokinetic resistance, whereby non-cycling cells resist the effects of topo II agents, may involve this type of resistance.
(d) A qualitative change in the topoisomerase II enzyme, a result either of a switch in gene expression (there are two genes for topoisomerase II and one is normally dominant) or of a mutation in the gene, or of a change in modification of the enzyme after it has been synthesised. This results in a change in drug-target interaction. The qualitative change is accompanied by a differential change in sensitivity: in general, cells become highly resistant to amsacrine, moderately resistant to etoposide and doxorubicin, and, we have ascertained, minimally resistant to DACA.

Investigation of the cytotoxicity of DACA under conditions of continuous drug exposure in a variety of human and mouse cell lines and in a panel of 60 human cell lines revealed IC₅₀ values (defined as drug con-centration required over approx. 5 cell doubling times for the reduction of the final cell culture density by 50%) ranging from 0.09 µM to 3.4µM, and a mean IC₅₀ value for human cell lines of 1.3µM. The latter value compared with 2 µM for the 4-pyridyl-quinoline analogue, 0.76 µM for amsacrine, 0.1 µM for the amsacrine analogue 4'-(9-[4-[N-methylcarboxamido]-5-methyl]-acridinylamino)-methanesulphon-m-anisidide ("CI-921") and 81 µM for etoposide. Whereas the patterns of cytotoxicity of amsacrine, CI-921 and etoposide in the human cell line panel were very similar, those of DACA and its pyridoquinoline analogue were quite different, suggesting differences in mode of action.

A multidrug resistant subline of P388 murine leukaemia (P/ACTD) was tested for sensitivity to DACA. This line was cross-resistant to actinomycin D, doxo-rubicin, mitoxantrone, etoposide and vincristine. Its resistance to vincristine was overcome by the presence of verapamil (10 µM). It stained for the presence of P-glycoprotein, consistent with the presence of transport-mediated multidrug resistance. This line was sensitive to DACA in vitro and in vivo, suggesting that DACA may be useful in at least some types of multidrug resistance.

DACA was also able to overcome, to a large extent, other mechanisms of multidrug resistance, as demonstrated in a series of sublines of Jurkat leukaemia cells which were highly resistant to amsacrine, etoposide and doxorubicin. Two of these lines had been selected for resistance to amsacrine, and were more than 100-fold cross-resistant to amsacrine but only 2- to 4-fold cross-resistant to DACA. These lines exhibited resistance mechanisms which were distinguishable from transport-mediated multidrug resistance. We believe that this ability to overcome resistance mechanisms accounts for the different IC₅₀ patterns observed with the human cell line panels.

It is apparent that in many tumours, regrowth during therapy is associated with resistance. The type of resistance is not yet properly characterised, but if it involves the mechanisms discussed above, DACA may be useful for second time treatment, especially in the divided dose regime mentioned above.

The use of all the above compounds and combinations in the treatment of sarcoma and of lung, breast, ovarian and testicular cancer and of melanoma and advanced colon cancer and brain tumours should especially be mentioned.

The use of compounds of the general formula I' to treat colon tumours has been suggested previously, but there has been no evidence of their suitability for this treatment and there has been no indication that they are effective in test systems even with delay of initiation of treatment beyond day 2 or 3 after tumour implantation, as is usual in tests. There has also been no disclosure of high activity against such tumours. Such high activity would not have been expected since the most closely structurally related topo II inhibitor, amsacrine, is inactive.

An initial experiment against advanced colon 38 tumour (on day 11 after implantation), using the same schedule of administration as used for Lewis lung (3 injections at 4-day intervals) gave only a modest growth delay (4 days).

However, by adjustment of the administration schedule of DACA, growth delay of the advanced colon 38 tumour (5-8 mm in diameter) was increased to more than 21 days. Thus, while intermittent schedules (270 mg/kg q7 days x 3; 400 mg/kg q7 days x 3) provided only modest growth delays (3 days and 7 days, respectively), repeated injection schedules (4 injections at 30 minute intervals; 180 + 120 + 120 + 120 µmol/kg,q7 days x 3) provided a 21 day growth delay. Such results were completely unexpected.

We have found that a low drug concentration for a long time (for example 6 hours) is much more toxic than a high concentration for a correspondingly shorter time. We believe these unusual "self-inhibitory" properties of DACA may be of help in the new divided dose administration strategy. Because DACA diffuses more slowly in solid tumours such as colon tumours, than in normal tissues, peak drug concentrations in tumours are lower than in normal tissues: an obvious disadvantage. However, because, as we have found, higher concentrations of DACA are less inhibitory than lower ones, the adjustment of the dosing strategy may provide partial protection of normal tissues.

The second and optional further administrations of the constituent doses of the divided-dose schedule of the present invention are at substantially more frequent intervals than are those of individual doses in conventional therapy - monthly, weekly or daily, for example - differing, for example, by an order of magnitude.

The second administration may be commenced, for example, at least 30 minutes, e.g. at least 45 min or at least 1 hr, but up to 8 hr, after commencement of the first administration; intervals of 30 min and of up to 1 hr, up to 90 min, up to 2 hr, up to 3 hr, up to 4 hr, up to 5 hr or up to 6hr should be mentioned. Any third or further administration may also, for example, be commenced at least 30 min, e.g. at least 45 min or at least 1 hr, and up to 8 hr after the beginning of the previous administration. As with the first interval, intervals of 30 min, and of up to 1 hr, up to 90 min, up to 2 hr, up to 3 hr, up to 4 hr, up to 5 hr or up to 6 hr should be mentioned. In the case of three or more administrations the intervals between two successive administrations may be the same or different.

Each constituent dose of the divided dose of the present invention may be administered by whatever method of administration is appropriate, for example by intravenous infusion over a period or by administration all at once; the amount of active ingredient in the different constituent doses may be the same or different. The or each interval of non-administration may be the same or different, for example in the range of from 15 mins to 8 hr, usually at least 30 min and often at least 1 hr and, for example, up to 6 hr, for example 30 min to 5 hr or 30 min to 4 hr; intervals of up to 2 hr, e.g. up to 1 hr, including 45 min or 30 min, should be mentioned.

There may, for example, be 2, 3, 4, 6, 8 or more administrations in a divided dose; these administrations may, for example, be made over a period in the range of from 30 min to 8 hr or longer, more usually up to 6 hr, for example up to 4 hr. Administration by the divided dose schedule may, if desired, be repeated once or more, after a suitable interval; such interval may be lengthy as in conventional therapy or may be shorter as in the present invention.

Thus the active ingredient may be administered over a period in the range of from 30 min to 8 hr, more usually from 1 hr to 8 hr, or more, the period depending on the interval(s) between individual constituent doses/repeats, on the number of doses and on the duration of each administration.

Thus, DACA or other acidine carboxamide of the general formula I', or salt or N-oxide thereof, may be administered, for example, in a divided dose over a period of up to 8 hours, for example up to 6 hours, advantageously up to 5 hours, e.g. up to 4 hours, for example 2 to 4 hours, followed by a rest, for example for 3 to 4 weeks. The dose may be divided into two to four administrations over the 2 to 4 hour or other administration period, and the first dose may be larger than the others, that is, as a loading dose; administrations may be given intravenously. For example, a short-term intravenous infusion of 10 to 30 minutes (for example 15 minutes) may be used, followed by a further such infusion after, for example, 1 hour. This schedule differs from that normally used for other cytotoxic agents, which involves periods of intravenous infusion administered daily, for example for 3 to 7 days, or long-term intravenous infusion over a number of days, for example for a week.

The first constituent dose is advantageously close to the maximum tolerable dose; that is, the maximum plasma level reached after the administration is close to the maximum that can be tolerated. Dosage schedules in which the second administration is made when or after the level of active ingredient in the blood has begun to fall, and optionally one or more further administrations are made also when or after the then level of active ingredient has begun to fall should be mentioned. Thus the rate of administration of the total dose over the administration period is not uniform, and the split doses provide cyclic peaks/troughs in plasma levels while giving lower steady-state concentrations in tumour tissue. Dosage schedules in which each trough for the plasma level is, for example, no less than 5% of the original maximum and each peak in the plasma level is, for example, up to 120% of the original maximum should especially be mentioned. Each trough for the plasma level may, for example, be in the range of from 5 to 50% of the original maximum, and each peak for the plasma level may, for example, be in the range of from 90 to 120% of the original maximum.

The second constituent dose may, for example, be administered when the plasma level of the drug has dropped to a value in the range of from 5 to 30% of the maximum level reached after administration of the first constituent dose, and any subsequent dose may, for example, be administered when the plasma level has dropped to a value in the range of from 10 to 50% of the maximum level reached after the immediately preceding administration.

We have found that DACA also has activity against melanoma cell lines and human tumour xenografts of these lines, and it is believed that this activity is improved by the same dosing strategy mentioned above.

The cytotoxicity of DACA was assessed in a panel of primary human melanoma cultures derived from fresh surgical melanoma specimens. IC₅₀ values ranged from 0.2 to 1.5 µM, and a feature of the data was the ability of DACA to kill much higher proportions of cells (>99%) in some cultures, as compared to a maximum of 90% for etoposide.

A further experiment was carried out using human melanoma line, implanted subcutaneously in nude (athymic) mice. Treatment was started when the tumours were 4-7 mm in diameter. DACA was administered ip as a divided dose (2 x 100 mg/kg body weight at 0 and 60 min) and a second similar administration (2 x 100 mg/kg) was given after 7 days. A growth delay of 30 days was obtained.

The positive results achieved by DACA in these treatments is surprising since melanoma is more difficult to treat with chemotherapy than are other forms of tumour.

Moreover, Berger et al. (Berger D.P., Winterhalter B.R., Flebig H.H., "Conventional chemotherapy" in "The Nude Mouse in Oncology Research", 1st ed. London: CRC Press, 1991, 165-84, ed. Boven and Winograd) states that melanoma xenografts are resistant to treatment by doxorubicin and etoposide, so activity by a drug in this class is completely unexpected. A summary of the activity of various other agents against subcutaneous melanoma xenografts growing in nude mice is given by Berger et al. as follows:

| Drug | Percentage of xenografts responding |
|---|---|
| Topo II inhibitors | |
| Doxorubicin | 5% (total of 5 studies) |
| Etoposide | 0% (2 studies) |

| Other drugs | |
|---|---|
| Bleomycin | 0% |
| Cisplatin | 13% |
| Cyclophosphamide | 11% |
| Dacarbazine | 17% |
| 5-Fluorouracil | 14% |
| Methotrexate | 7% |
| Mitomycin C | 32% |
| Vinblastine | 10% |
| Vincristine | 43% |

In pharmacokinetic studies using radioactive (tritium-labelled) DACA high levels of active ingredient have been found in all tissues, including brain, with a long elimination t_{1/2} of 37-176 h. As determined by HPLC, the tissue concentrations of DACA 1 h after intraperitoneal administration of drug (400 µmol/kg) were 45, 185, 139, and 57 µmol/kg in brain, liver, kidney and heart, respectively. The corresponding AUC values (AUC = area under the plasma concentration-time curve) were 218, 547, 492 and 147 µmol.h/l, respectively, as compared to the plasma AUC of 26.6 µmol.h/l. DACA showed relatively high rates of passage across the blood brain barrier. We believe that administration of DACA at the new divided dose-high constituent dose frequency regime mentioned above will be helpful in combating brain tumours. With the exception of nitrosoureas, few of the antitumour agents currently in use possess the physicochemical properties required for adequate penetration of the blood-brain barrier (Greig M.H. (1987), Cancer Treat. Rep. **11**: 157).

We also propose the use of DACA and related compounds with a "rescue" treatment with a second drug which by itself is not an active agent but which displaces DACA or the other compound from the DNA. This DNA-binder, or chemoprotector, should have a lower intrinsic toxicity and less efficient tissue distribution properties than the cytotoxic agent, thus sparing rapidly-growing and highly vascularised normal tissues such as bone marrow from cytotoxic effects. Use of the new schedule of administration of DACA or other compound of the general formula I or a physiologically tolerable acid addition salt or 1-N-oxide thereof, combined with "rescue" treatment with a chemoprotector, should especially be mentioned. Timing of administration of the chemoprotector will depend on the pharmacokinetics of DACA or other drug used. The chemoprotector may, for example, be administered at the same time as or up to 30 minutes after one or more of the constituent doses of a divided dose of that drug; by such means doses of, for example, 200 mg/kg or even 300 mg/kg of DACA - doses which are normally toxic - may be possible.

The following Examples illustrate the invention.

### Examples

### Reference Example 1

### Activity of DACA against cultured multidrug resistant human leukaemia cells

### Materials and Methods

Acridine carboxamide hydrochloride, synthesised in the Cancer Research Laboratory (Atwell G.J., et al., J.Med. Chem. 1987, **30**, 664-669), and amsacrine isethionate, obtained from the Parke-Davis Division of the Warner-Lambert Company, Ann Arbor, USA, were dissolved in 50% v/v aqueous ethanol to make stock solutions of 2-5 mmol/l and stored at -20°C. Other cytotoxic drugs were available either from the NCI repository (Monks A., et al., J. Natl. Cancer Inst. 1991, **83**, 757-766) or were obtained as described in Marshall E.S., et al., J. Natl. Cancer Inst. 1992, **84**, 341-344 and Finlay, et al., Eur. J. Cancer din. Oncol. 1986, **22**, 655-662. Cell lines were from the NCI repository except for MM-96 (Dr. R. Whitehead, Ludwig Institute, Melbourne, Australia), FME (Dr. K.M. Tveit, Norwegian Radium Hospital, Oslo, Norway) and Jurkat normal and multidrug-resistant lines (Dr. K. Snow and Dr. W. Judd, Department of Cellular and Molecular Biology, University of Auckland). Melanoma tissue was obtained from patients with pathologically confirmed metastatic and recurrent melanomas under Auckland Hospital Ethical Committee guidelines. Cells were released by digestion of tissue (at 50 mg.ml⁻¹) with collagenase (1 mg.ml⁻¹) and DNAase (50 µg.ml⁻¹) with continuous stirring at 37°C for 1 to 2 hours, and cultured as previously described (Marshall E.S., et al., J. Natl. Cancer Inst. 1992, **84**, 341-344).

Tumour cell lines were cultured in 96-well plates. Growth of NCI cell lines was assessed using sulpho-rhodamine B staining (Skehan P., et al., J. Natl. Cancer Inst. 1990, **82**, 1107-1112), that of the leukaemia lines with (4,5-dimethylthiazole-2-yl)-2,5-diphenyltetrazolium (MTT) staining (Mosmann T., J. Immun. Methods 1983, **65**, 55-63) and that of the primary human tumour material by ³H-thymidine incorporation (Marshall E.S., et al., J. Natl. Cancer Inst. 1992, **84**, 341-344). Primary tumour material was cultured in 96-well plates in which the wells were coated with agarose to inhibit selectively the growth of normal cells. (Marshall E.S., et al., J. Natl. Cancer Inst. 1992, **84**, 341-344). Primary cultures were incubated at 37°C in sealed perspex boxes containing a humidified atmosphere of 5% CO₂ and 5% O₂ in nitrogen for 7 days. 5-Methyl-[³H]-thymidine (20 Ci.mmol⁻¹; 0.04 µCi per well), thymidine and 5-fluorodeoxyuridine (each at final concentrations of 0.5 µM) were added in medium to cultures (20 µl per well) 24 h before terminating the cultures. Cells were aspirated on to glass fibre filters using a multiple automated sample harvester (LKB Wallac OY Beta Harvester). The filter discs were washed for 15 seconds with water, dried, and the amount of tritium retained quantified by liquid scintillation.

IC₅₀ values were defined as in Paull K.D., et al., J. Natl. Cancer Inst. 1989, **81**:1088-1092, where growth, as indicated by staining or thymidine incorporation, corresponded to 50% of that of the control cultures. DELTA values were determined for groups of logarithmic IC₅₀ values as deviations from the mean, with positive DELTA values representing higher drug sensitivity relative to the mean. Variances of DELTA values were expressed as standard deviations in log₁₀ units. Comparison of DELTA values was made using Pearson correlation coefficients. Resistance factors were defined as the ratios of IC₅₀ values between the resistant line and the parent line. Statistical evaluation was performed either with NCI programmes, with RS/1 software (BBN Research Systems, Cambridge, MA, USA), or with Sigmaplot (Jandel Scientific, San Rafael, CA, USA).

### Results

The effect of DACA on the growth of cultured cells was assessed by continuous drug exposure. DACA inhibited the growth of two human Jurkat T cell leukaemia lines, one diploid (L) and the other tetraploid (B1), with IC₅₀ values each of 380 nM. These values were similar to those of other human leukaemia cell lines, which ranged from 290 to 760 nM (CEM-CCRF, 410 nM; MOLT-4, 290 nM; Daudi, 400 nM; Raji, 370 nM, U937, 590 nM; HL-60, 620 nM; K-562, 760 nM). Four multidrug-resistant cell lines, developed from JL and JB1 by in vitro exposure to increasing concentrations of doxorubicin (L_{D} and B1_{D}) or amsacrine (L_{A} and B1_{A}) (Finlay G.J., et al., J. Natl. Cancer Inst. 1990, **82**, 662-667, Snow K., et al., Br. J. Cancer 1991, **63**, 17-28) were tested.

DACA was compared with six other drugs including four topo II poisons, doxorubicin, mitozantrone, etopo-side and amsacrine. Resistance factors for the topo II poisons were consistently higher than those for DACA (Table 1). In contrast, the topoisomerase I poison camptothecin showed no cross resistance, and the mitotic inhibitor vincristine showed a different pattern of resistance with the B1_{D} line having the highest resistance (Table 1).

**Table 1**

| Drug-resistant Jurkat Leukaemia sublines. | | | | | | | |
|---|---|---|---|---|---|---|---|
| | Resistance factors | | | | | | |
| | doxorubicin | mitoxantrone | etoposide | amsacrine | DACA | camptothecin | vincristine |
| L_{A} | 3.8 | 42 | 11 | 130 | 2.0 | 1.0 | 1.5 |
| L_{D} | 16 | 160 | 93 | 110 | 2.5 | 0.97 | 3.6 |
| B1_{A} | 11 | 59 | 22 | 240 | 3.9 | 0.48 | 2.0 |
| B1_{D} | 15 | 8.4 | 83 | 8.8 | 1.9 | 0.86 | 10 |

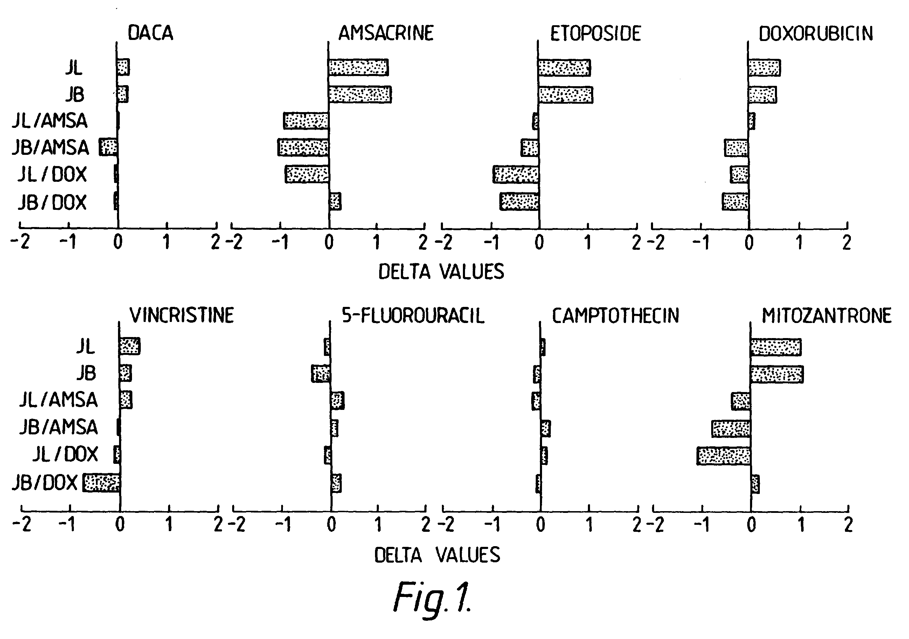

One method of providing a visual comparison of the patterns of resistance is to plot DELTA values (Paull K.D., et al., J. Natl. Cancer Inst. 1989, **81**, 1088-1092) where the differences in bar lengths are used as a measure of relative resistance. Figure 1 shows a comparison of DELTA values in log mean graph format for DACA, amsacrine, etoposide, doxorubicin, vincristine, 5-fluorouracil, camptothecin and mitozantrone for the panel of multidrug resistante jurkat leukaemia lines using MTT staining; JL/AMSA and JB/AMSA were selected for resistance to amsacrine and JL/DOX and JB/DOX were resistant to doxorubicin. DACA clearly shows a pattern distinct from doxorubicin.

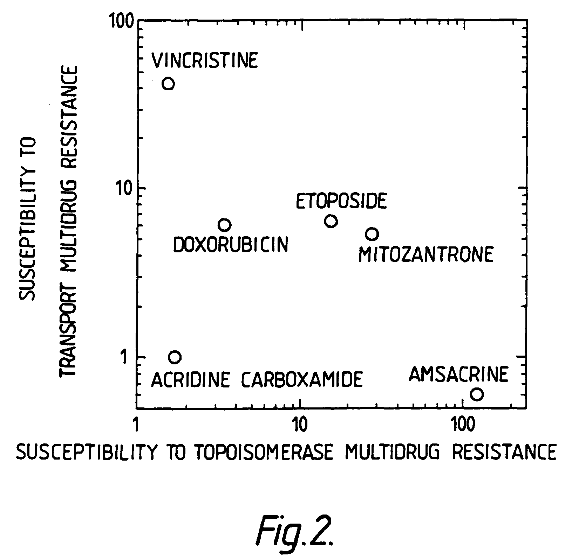

A second method of comparing agents is to plot resistance factors for one of the lines against another. Since the Jurkat lines exhibited predominantly "altered topoisomerase" resistance (Finlay G.J., et al., J. Natl. Cancer Inst. 1990, **82**, 662-667, Sugimoto Y., et al., Cancer Res. 1990, **50**, 7962-7965), the resistance factors for one of these (L_{A}) was plotted versus the resistance factors for a P-glycoprotein positive multidrug resistant P388 Leukaemia line (P/DACT) which exhibits transport resistance (Baguley B.C., J. Natl. Cancer Inst. 1990, **82**, 398-402). The results (Figure 2) indicate that DACA is unique when compared to other topo II agents in that it is able to overcome two different multidrug resistance mechanisms. Qualitatively similar graphs are obtained when the resistance factors of the other resistant Jurkat lines are plotted on the abscissa, or those from a P-glycoprotein positive, vinblastine resistant human leukaemia line (CEM/VLB₁₀₀) (Qian X., Beck W.T., Cancer Res. 1990, **50**, 1132-1137) are plotted on the ordinate.

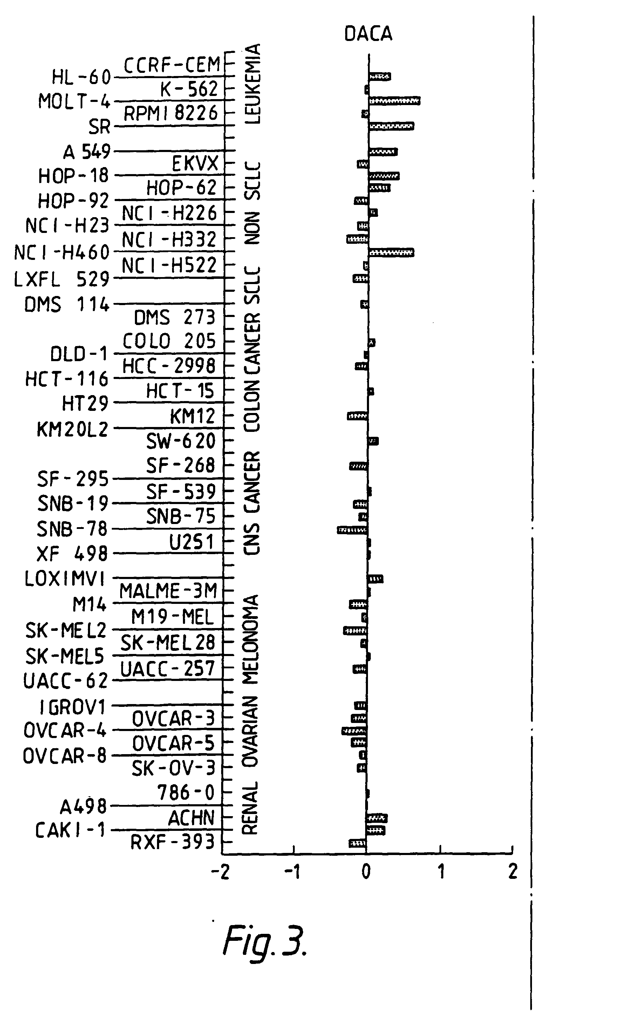

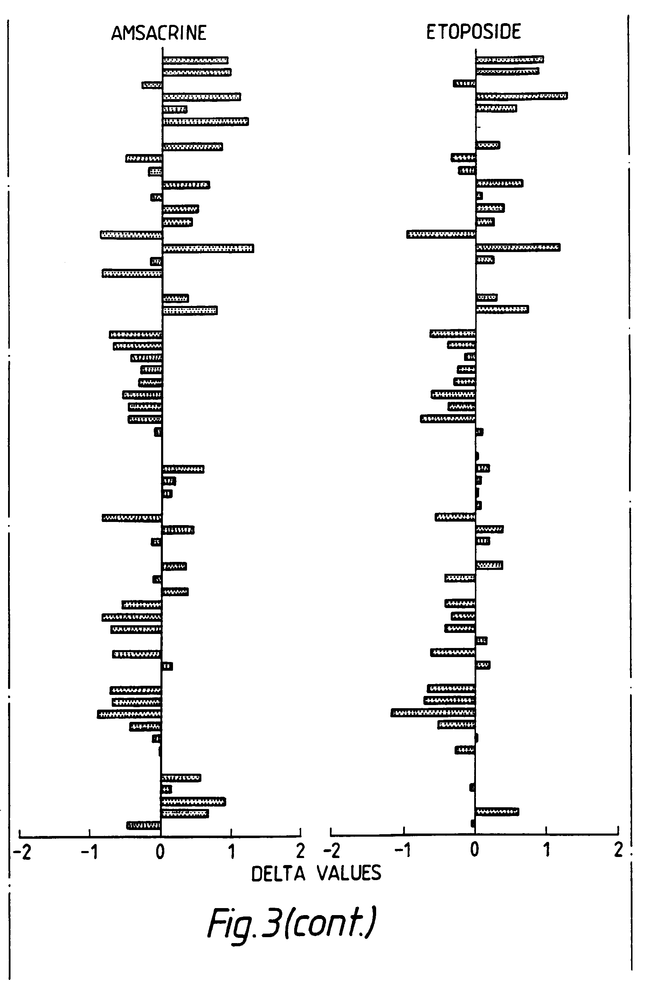

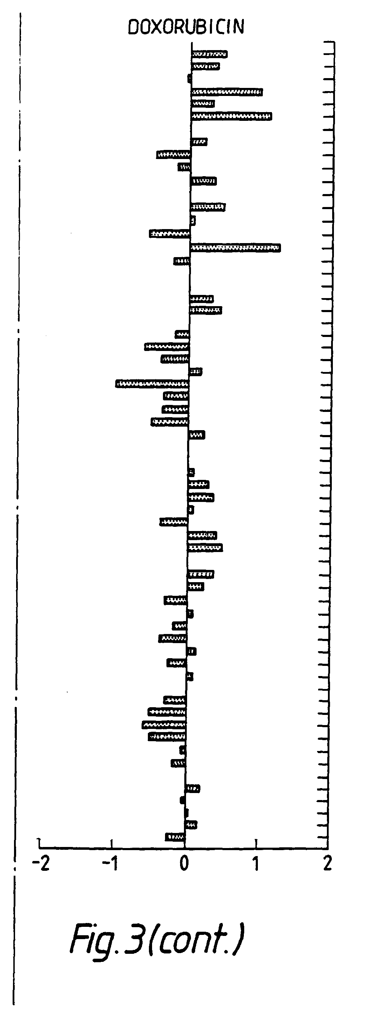

DACA was also compared with three other topo II agents using a panel of cell lines (data provided by Dr. Ken Paull from the National Cancer Institute, USA) encompassing a number of tumour types, and using protein staining. The mean IC₅₀ for DACA was 2,100 nM, as compared with amsacrine (520 nM), etoposide (21,000 nM) and doxorubicin (140 nM). The results, presented as DELTA plots, are compared with corresponding plots for three other topoisomerase II poisons in Figure 3. The variance of DELTA values was considerably smaller for DACA (0.24 units) than it was for amsacrine (0.61 units) etoposide (0.55 units) or doxorubicin (0.44 units). The differences in DELTA values for amsacrine, etoposide and doxorubicin for primary human cultures imply that intrinsic resistance mechanisms exist and are partially overcome with DACA.

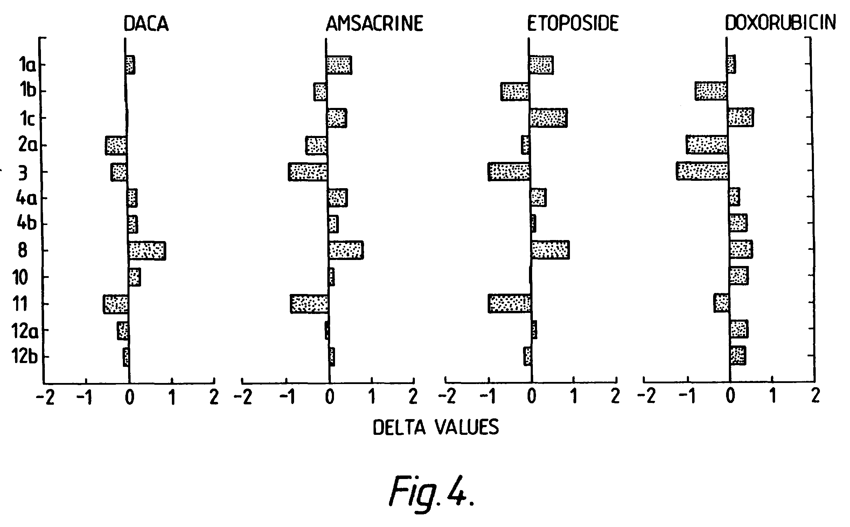

DACA was also compared in a series of 12 primary melanoma cultures. Tissue was excised from human malignant melanomas and cultured using a modified 96-well assay system in which the cells were cultured on agarose and assayed for proliferation using the ³H-thymidine incorporation assay as described in Marshall E.S., et al., J. Natl. Cancer Inst. 1992, **84**, 341-344. The mean IC₅₀ for DACA was 590 nM, as compared with amsacrine (128 nM), etoposide (2,200 nM) and doxorubicin (56 nM). DELTA values for DACA, amsacrine, etoposide and doxo-rubicin are compared in Figure 4. The variance of DELTA values was smaller for DACA (0.39 units) than for amsacrine (0.54 units), etoposide (0.66 units) or doxorubicin (0.63 units). The differences in DELTA values for amsacrine, etoposide and doxorubicin for primary human cultures again imply that intrinsic resistance mechanisms exist and are partially overcome with DACA.

### Example 1

### Activity of DACA against advanced colon 38 and melanoma in mice

### Materials and Methods

Colon 38 carcinoma was obtained from the Mason Research Institute (Worchester, MA, USA) and was grown in BDF₁ hosts. Tumour fragments (1 mm³) were implanted subcutaneously in anaesthetised mice. Tumours had grown to the appropriate size 9 days after implantation. A melanoma tumour line (WADH) was developed in the Cancer Research Laboratory. Tumour cells were grown in culture and 1 x 10⁶ cells were implanted intradermally into the flank of nude (C57BI/J genetic background) mice. Mice were grown under sterile surroundings until tumours were of appropriate size.

Tumours were measured 3x (colon 38) or 2x (xeno-graft) weekly with callipers and tumour volumes calculated as 0.52a²b, where a and b were the minor and major tumour axes. Tumour growth delays were measured at a time when tumour volumes of treated and control animals had increased by 4-fold.

### Results

The effects of five compounds,
(i) DACA
(ii) N-[2-(dimethylamino)ethyl]-9-methoxyphen-azine-1-carboxamide (compound 28 of J. Med. Chem., 1987, **30**, 843-851),
(iii) N-[2-(dimethylamino) ethyl]-2-phenylquino-line-8-carboxamide (compound 6 of J. Med. Chem. 1988, **31**, 1048-1052),
(iv) N-[2-(dimethylamino)ethyl]-2-(4-pyridyl)-guinoline-8-carboxamide (compound 18 of J. Med. Chem., 1989, **32**, 396-401), and
(v) N-[2-(dimethylamino)ethyl]-9-bromo-dibenzo-[1,4]-dioxin-1-carboxamide (compound 20 of J. Med. Chem. 1992, **35**, 258-266)
   on the growth of advanced colon 38 tumours in mice was investigated by implanting tumour fragments subcutaneously and allowing them to grow until they had reached a diameter of 5-8 mm.

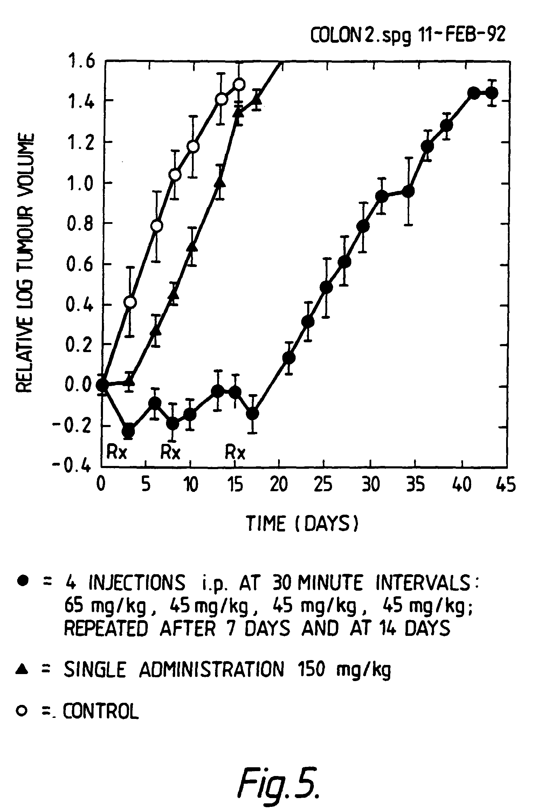

I.p. treatment of mice with a single maximum tolerated dose of DACA (150 mg/kg body weight), a treatment which was known to induce cures of intravenously implanted Lewis lung tumours (Finlay G.J., Baguley B.C., Eur. J. Cancer Clin. Oncol. 1989, **25**, 271-277) caused only a slight growth delay (5 days; Figure 5). However, when a divided dose (200 mg/kg) was administered over a period of 0.5 - 4 hours, greater delays were unexpectedly observed (Table 2). Repetition of these divided doses provided a substantial growth delay (23 days; Figure 5) which was longer than that obtained with the maximum tolerated dose of amsacrine (2 days), cyclophosphamide (6.5 days) or 5-fluorouracil (13 days).

**Table 2**

| Tumour growth delays (colon 38) treated with DACA | | | |
|---|---|---|---|
| Total | dose | Schedule | Growth delay (days) |
| 100 | ip | single dose (SD) | 4 |
| 150 | ip | SD | 5 |
| 150x3 | ip | SD every week x 3 | 7 |
| 150 | ip | 2 doses, 0, 60 min | 5 |
| 200 | ip | SD | toxic |
| 200 | ip | 2 doses, 0, 30 min | 7 |
| 200 | ip | 2 doses, 0, 60 min | 10, 12 (2 expts) |
| 200 | ip | 2 doses, 0, 24 hours | 6 |
| 200 | ip | 4 doses, 0, 30, 60, 90 min | 6 |
| 200 | iv | 1 hour infusion | 7 |
| 200 | iv | 3 hour infusion | 6.5 |
| 200x3 | ip | (4 doses, 0, 30, 60, 90)x3 | 23 |
| Note: the 4 dose schedule was 65 + 45 + 45 + 45 mg/kg | | | |

The other compounds were each administered by ip injection in two doses one hour apart at a dose (for each injection) of 100mg/kg for the phenazine, 75 mg/kg for the phenylguinoline, 50mg/kg for the pyridylquinoline and 100 mg/kg for the dibenzodioxin. The growth delays obtained are shown in Table 3, together with the result obtained with DACA at the same schedule of administration.

**Table 3**

| Tumour growth delays (colon 38) treated with tricyclic carboxamide derivatives. | | | | |
|---|---|---|---|---|
| Compound | Dose each injection | Route | Schedule | Growth delay (days) |
| (i) | 100mg/kg | ip | 2 doses, 0, 60 min | 10,12 (2 expts) |
| (ii) | 100mg/kg | ip | 2 doses, 0,60 min | 5,5 (2 expts) |
| (iii) | 75mg/kg | ip | 2 doses, 0,60 min | 8.5 |
| (iv) | 50mg/kg | ip | 2 doses, 0,60 min | 5 |
| (v) | 100mg/kg | ip | 2 doses, 0,60 min | 7 |

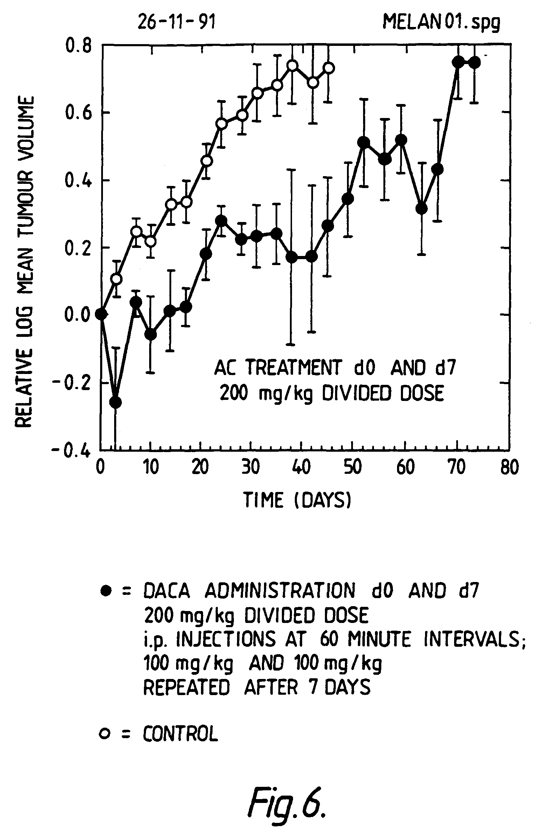

A further experiment was carried out using human melanoma line, implanted subcutaneously in nude (athymic) mice using an inoculation of one million cells of a human melanoma cell line designated WADH. Treatment was started when the tumours were 4-7 mm in diameter. DACA was administered ip as a divided dose (2 x 100 mg/kg body weight at 0 and 60 min) and a second similar administra-tion (2 x 100 mg/kg) was given after 7 days. A growth delay of 30 days was obtained (Figure 6).

## Claims

1. The use of a tumour-inhibiting compound which is an acridine carboxamide of the formula (I') 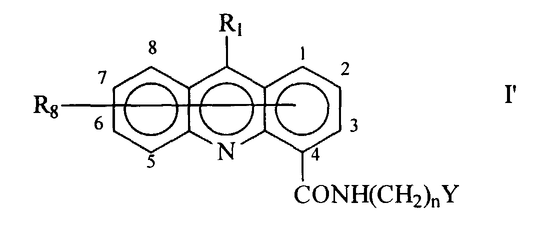
wherein R₁ is H, CR₃ or NHR₀,
where Rₒ is H, COCH₃, SO₂CH₃, COPh, SO₂Ph or C₁-C₄ alkyl which is unsubstituted or substituted by hydroxy, C₁-C₅ alkoxy or amino;
n is 2;
R₈ is H or is one or two substituents selected from CH₃, OCH₃, halogen, CF₃, NO₂, NH₂, NHCOCH₃, and NHCOOCH₃ at positions 1-3 and 5-8; and
Y is C(NH)NH₂, NHC(NH)NH₂, or NR₄R₅, wherein each of R₄ and R₅ is H or C₁-C₅ alkyl which is unsubstituted or substituted by hydroxyl or amino; or a physiologically tolerable acid addition salt or N-oxide thereof; in the manufacture of a medicament for the treatment of tumours by a divided-dose schedule comprising a first administration and a second administration of the sai d compound, wherein the second administration commences between 15 minutes and 24 hours after commencement of the first administration.

2. A use according to claim 1 wherein the divided dose schedule comprises at least 2 administrations of drug over a period of from 30 minutes to 8 hours.

3. A use according to claim 1 or 2, wherein the divided dose schedule comprises at least 2 administrations of drug over a period of from 30 minutes to 6 hours.

4. A use according to any one of claims 1 to 3, wherein the divided dose schedule comprises at least 2 administrations of drug over a period of from 30 minutes to 4 hours.

5. A use according to any one of claims 1 to 4, wherein the schedule comprises 2 to 4 administrations of drug over a period of 3 to 4 hours.

6. A use according to any one of claims 1 to 5, wherein the administration is to a patient having multidrug resistant cancer.

7. The use of a tumour-inhibiting compound which is an acridine carboxamide of the formula (I') 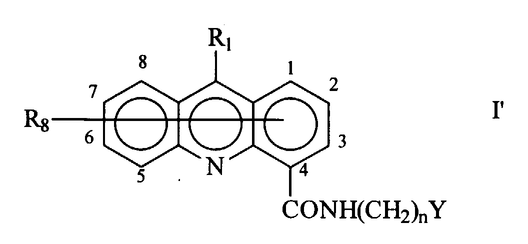
wherein R₁ is H, CH₃ or NHR₀,
where Rₒ is H, COCH₃, SO₂CH₃, COPh, SO₂Ph or C₁-C₄ alkyl which is unsubstituted or substituted by hydroxy, C₁-C₅, alkoxy or amino;
n is 2;
R₈ is H or is one or two substituents selected from CH₃, OCH₃, halogen, CF₃, NO₂, NH₂, NHCOCH₃, and NHCOOCH₃ at positions 1-3 and 5-8; and
Y is C(NH)NH₂, NHC(NH)NH₂, or NR₄R₅, wherein each of R₄ and R₅ is H or C₁-C₅ alkyl which is unsubstituted or substituted by hydroxyl or amino or a physiologically tolerable acid addition salt or N-oxide thereof; in the manufacture of a medicament for circumventing multidrug resistance in the treatment of tumours by a divided dose schedule comprising a first administration and a second administration of the said compound, wherein the second administration commences between 15 minutes and 24 hours after commencement of the first administration.

8. A use according to claim 7 wherein the divided dose schedule comprises at least 2 administrations of drug over a period of from 30 minutes to 8 hours.

9. A use according to claim 7 or 8 wherein the divided dose schedule comprises at least 2 administrations of drug over a period of from 30 minutes to 6 hours.

10. A use according to any one of claims 7 to 9 wherein the divided dose schedule comprises at least 2 administrations of drug over a period of from 30 minutes to 4 hours.

11. A use according to any one of claims 7 to 10 wherein the divided dose schedule comprises 2 to 4 administrations of drug over a period of 2 to 4 hours.

12. A use according to any one of claims 6 to 11, wherein the multidrug resistance is expressed by two or more multidrug resistance mechanisms.

13. A use according to any one of the preceding claims wherein the divided dose schedule comprises a third administration and optional further administrations of the said compound, each of the said third and further administrations commencing between 15 minutes and 24 hours after commencement of the previous administration.

14. A use according to any one of claims 1 to 13 wherein the administration includes administration of a second tumour-inhibiting compound that is not a topo-II inhibitor.

15. A use according to claim 14, wherein the two compounds are administered as separate or alternating courses.

16. A use according to claim 14 or claim 15, wherein the dose(s) or at least one of the constituent doses of the acridine carboxamide of the general formula I' or salt or N-oxide thereof and the second tumour-inhibiting compound are administered within 2 days of each other.

17. A use according to any one of claims 14 to 16, wherein the second tumour-inhibiting compound is cisplatin, cyclophosphamide, bleomycin, carboplatin, decarbazine, mitomycin C, melphalan, 5-fluorouracil, 5-fluorodeoxyuridine, methotrexate, taxol, vincristine, vinblastine, vindesine, tamoxifen, tissue necrosis factor, interleukin II or interferon.

18. A use according to any one of claims 1 to 17, wherein the administration is to a patient suffering from sarcoma, melanoma or lung, breast, ovarian, testicular or colon cancer or brain tumours.

19. A use according to claim 18, wherein administration is to a patient having melanoma, advanced colon cancer or a brain tumour.

20. A use according to any one of claims 1 to 19, wherein at least one of the constituent doses of the acridine carboxamide of the general formula I' or salt or N-oxide thereof is used with a further component which is a DNA-binding agent that reduces the host toxicity of the first component, this further component and the acridine carboxamide of formula I' or salt or N-oxide thereof being present together in the same formulation or in separate formulations for simultaneous or sequential administration.

21. A use according to any one of the preceding claims wherein the tumour-inhibiting compound is N-[2-(dimethyl-amino) ethyl] cridine-4-carboxamide.

## Patentansprüche

1. Verwendung einer Tumor inhibierenden Verbindung, welche ein Acridincarboxamid der Formel (I') ist 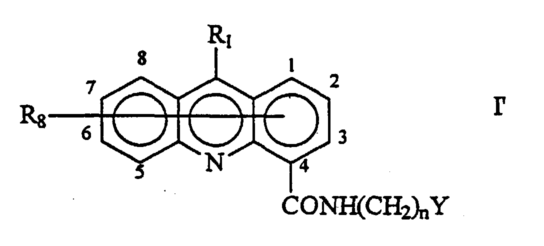
worin R₁ H ist, CH₃ oder NHR₀,
wobei R₀ H ist, COCH₃, SO₂CH₃, COPh, SO₂Ph oder C₁-C₄ Alkyl, welches unsubstituiert ist oder durch Hydroxy, C₁-C₅ Alkoxy oder Amino substituiert ist;
n ist 2;
R₈ ist H oder einer oder zwei Substituenten ausgewählt aus CH₃, OCH₃, Halogen, CF₃, NO₂, NH₂, NHCOCH₃ und NHCOOCH₃ an den Positionen 1-3 und 5-8; und
Y ist C(NH)NH₂, NHC (NH) NH₂, oder NR₄R₅, wobei jeder von R₄ und R₅ H ist oder C₁-C₅Alkyl, welches unsubstituiert ist oder durch Hydroxyl oder Amino substituiert; oder ein physiologisch verträgliches Säureadditionssalz oder N-Oxid davon; bei der Herstellung eines Medikamentes zur Behandlung von Tumoren nach einem Chargendosisplan, umfassend eine erste Verabreichung und eine zweite Verabreichung der Verbindung, wobei die zweite Verabreichung zwischen 15 Minuten und 24 Stunden nach Beginn der ersten Verabreichung beginnt.

2. Verwendung gemäß Anspruch 1, wobei der Chargendosispian wenigstens zwei Arzneimittelverabreichungen über einen Zeitraum von 30 Minuten bis 8 Stunden umfasst.

3. Verwendung gemäß Anspruch 1 oder 2, wobei der Chargendosispian wenigstens 2 Arzneimittelverabreichungen über einen Zeitraum von 30 Minuten bis 6 Stunden umfasst.

4. Verwendung gemäß einem der Ansprüche 1 bis 3, wobei der Chargendosisplan wenigstens 2 Arzneimittelverabreichungen über einen Zeitraum von 30 Minuten bis 4 Stunden umfasst.

5. Verwendung gemäß einem der Ansprüche 1 bis 4, wobei der Chargendosisplan 2 bis 4 Arzneimittelverabreichungen über einen Zeitraum von 3 bis 4 Stunden umfasst.

6. Verwendung gemäß einem der Ansprüche 1 bis 5, wobei die Verabreichung an einen Patienten mit gegen mehrere Arzneimittel resistentem Krebs erfolgt.

7. Verwendung einer Tumor inhibierenden Verbindung, welche ein Acridincarboxamid der Formel (I') ist 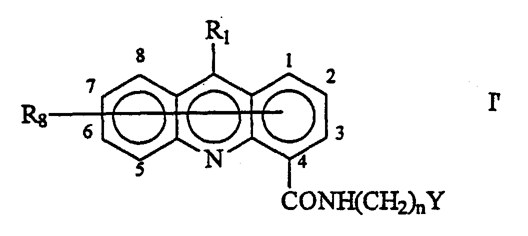
worin R₁ H ist CH₃ oder NHR₀,
wobei R₀ H ist, COCH₃, SO₂CH₃, COPh, SO₂Ph oder C₁-C₄ Alkyl, welches unsubstituiert ist oder durch Hydroxy, C₁-C₅ Alkoxy oder Amino substituiert;
n ist 2;
R₈ ist H oder eine oder zwei Substituenten, ausgewählt aus CH₃, OCH₃, Halogen, CF₃, NO₂, NH₂, NHCOCH₃ und NHCOOCH₃ an den Positionen 1-3 und 5-8; und
Y ist C (NH) NH₂, NHC (NH) NH₂ oder NR₄R₅, wobei jeder von R₄ und R₅ H ist oder C₁-C₅ Alkyl, welches unsubstituiert oder durch Hydroxyl oder Amino substituiert ist oder ein physiologisch verträgliches Säureadditionssalz oder N-Oxid davon; bei der Herstellung eines Medikamentes zur Umgehung einer Mehrfacharzneimittelresistenz bei der Behandlung von Tumoren mittels eines Chargendosispians, der eine erste Verabreichung und eine zweite Verabreichung der Verbindung umfasst, wobei die zweite Verabreichung zwischen 15 Minuten und 24 Stunden nach dem Beginn der ersten Verabreichung beginnt.

8. Verwendung gemäß Anspruch 7, wobei der Chargendosisplan wengistens 2 Arzneimittelverabreichungen über einen Zeitraum von 30 Minuten bis 8 Stunden umfasst.

9. Verwendung gemäß Anspruch 7 oder 8, wobei der Chargendosisplan wenigstens 2 Arzneimittelverabreichungen über einen Zeitraum von 30 Minuten bis 6 Stunden umfasst.

10. Verwendung gemäß einem der Ansprüche 7 bis 9, wobei der Chargendosisplan wenigstens 2 Arzneimittelverabreichungen über einen Zeitraum von 30 Minuten bis 4 Stunden umfasst.

11. Verwendung gemäß einem der Ansprüche 7 bis 10, wobei der Chargendosisplan 2 bis 4 Arzneimittelverabreichungen über einen Zeitraum von 2 bis 4 Stunden umfasst.

12. Verwendung gemäß einem der Ansprüche 6 bis 11, wobei die Mehrfacharzneimittelresistenz über zwei oder mehrere Mehrfacharzneimittelresistenzmechanismen Ausdruck findet.

13. Verwendung gemäß einem der vorhergehenden Ansprüche, wobei der Chargendosispian eine dritte Verabreichung und optional weitere Verabreichungen der Verbindung umfasst, wobei jede der dritten und weiteren Verabreichungen zwischen 15 Minuten und 24 Stunden nach dem Beginn der vorherigen Verabreichung beginnt.

14. Verwendung gemäß einem der Ansprüche 1 bis 13, wobei die Verabreichung eine Verabreichung einer zweiten Tumor inhibierenden Verbindung einschließt, die kein Tope-II Inhibitor ist.

15. Verwendung gemäß Anspruch 14, wobei die beiden Verbindungen separat oder altemierend verabreicht werden.

16. Verwendung gemäß Anspruch 14 oder 15, wobei die Dosis (Dosen) oder wenigstens eine der Bestandteil bildenden Dosen des Acridincarboxamids der allgemeinen Formel I' oder Salz oder N-Oxid davon und die zweite Tumor inhibierende Verbindung innerhalb von zwei Tagen verabreicht werden.

17. Verwendung gemäß einem der Ansprüche 14 bis 16, wobei die zweite, Tumor inhibierende Verbindung Cisplatin, Cyclophosphamid, Bleomyzin, Carboplatin, Decarbazin, Mitomyzin C, Melphalan, 5-Fluoruracil, 5-Flourodeoxyuridin, Methotrexat, Taxol, Vincristin, Vinblastin, Vindesin, Tamoxifen, Gewebenekrosefaktor, Interleukin II oder Interferon ist.

18. Verwendung gemäß einem der Ansprüche 1 bis 17, wobei die Verabreichung an einen Patienten erfolgt, der an einem Sarkom, Melanom, oder Lungen-, Brust-,Eierstock-, Hoden- oder Darnikrebs leidet oder an Gehirntumoren.

19. Verwendung gemäß Anspruch 18, wobei die Verabreichung an einen Patienten mit einem Melanom, fortgeschrittenem Darmkrebs oder einem Gehimtumor erfolgt.

20. Verwendung gemäß einem der Ansprüche 1 bis 19, wobei wenigstens eine der Bestandteil bildenden Dosen des Acridincarboxamids der allgemeinen Formel I' oder des Salzes oder des N-Oxids davon in Verbindung mit einem weiteren Bestandteil verwendet wird, der ein DNA-Bindungsagens ist, welches die Wirtstoxizität des ersten Bestandteiles verringert, wobei dieser weitere Bestandteil und das Acridincarboxamid der Formel I' oder das Salz oder das N-Oxid davon zusammen in derselben Formulierung vorliegen oder in getrennten Formulierungen zur gleichzeitigen oder sequentiellen Verabreichung.

21. Verwendung gemäß einem der vorhergehenden Ansprüche, wobei die Tumor inhibierende Verbindung N-[2-(Dimethyl-amino)ethyl]acridin-4-carboxamid ist.

## Revendications

1. Utilisation d'un composé acridine carboxamide qui inhibe les tumeurs et qui a la formule (I') suivante 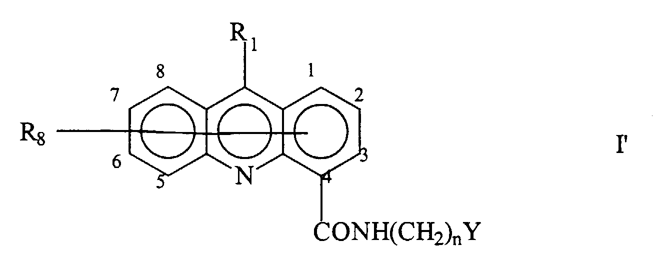
dans laquelle R₁ représente un atome H, un groupement CH₃ ou NHR₀,
où Rₒ représente un atome H, un groupement COCH₃, SO₂CH₃, COPh, SO₂Ph ou un alkyle en C₁-C₄ qui est non-substitué ou substitué par un groupement hydroxy, alkoxy en C₁-C₅ ou amino;
n est 2;
R₈ représente un atome H ou un ou deux substituants sélectionnés parmi les groupements CH₃, OCH₃, halogène, CF₃, NO₂, NH₂, NHCOCH₃, et NHCOOCH₃ aux positions 1-3 et 5-8 ; et
Y représente un groupement C(NH)NH₂, NHC(NH)NH₂, ou NR₄R₅, dans lequel chacun des substituants R₄ et R₅ représente un atome H ou un groupement alkyle en C₁-C₅ qui est non-substitué ou substitué par un groupement hydroxyle ou amino ; ou un sel acide ou un N-oxyde de celui-ci qui est physiologiquement tolérable ; dans la production d'un médicament utile dans le traitement des tumeurs qui est un schéma posologique à doses fractionnées ; ce traitement comporte une première administration et une seconde administration dudit composé, la seconde administration commençant entre 15 minutes et 24 heures après le début de la première administration.

2. Utilisation conformément à la revendication 1 dans laquelle le schéma posologique à doses fractionnées comprend au moins 2 administrations du médicament sur un intervalle allant de 30 minutes à 8 heures.

3. Utilisation conformément à la revendication 1 ou 2 dans laquelle le schéma posologique à doses fractionnées comprend au moins 2 administrations du médicament sur un intervalle allant de 30 minutes à 6 heures.

4. Utilisation conformément à l'une quelconque des revendications 1 à 3 dans laquelle le schéma posologique à doses fractionnées comprend au moins 2 administrations du médicament sur un intervalle allant de 30 minutes à 4 heures.

5. Utilisation conformément à l'une quelconque des revendications 1 à 4 dans laquelle le schéma posologique comprend entre 2 et 4 administrations du médicament sur un intervalle allant de 3 heures à 4 heures.

6. Utilisation conformément à l'une quelconque des revendications 1 à 5, dans laquelle le patient qui reçoit le médicament est atteint d'un cancer qui présente une multidrogue résistance.

7. Utilisation d'un composé acridine carboxamide qui inhibe les tumeurs et qui a la formule (I') suivante 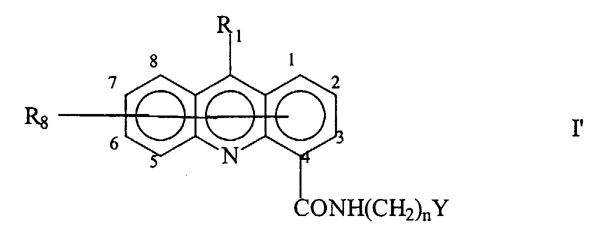
dans laquelle R₁ représente un atome H, un groupement CH₃ ou NHR₀,
où Rₒ représente un atome H, un groupement COCH₃, SO₂CH₃, COPh, SO₂Ph ou un alkyle en C₁-C₄ qui est non-substitué ou substitué par un groupement hydroxy, alkoxy en C₁-C₅ ou amino;
n est 2;
R₈ représente un atome H ou un ou deux substituants sélectionnés parmi les groupements CH₃, OCH₃, halogène, CF₃, NO₂, NH₂, NHCOCH₃, et NHCOOCH₃ aux positions 1-3 et 5-8 ; et
Y est un groupement C(NH)NH₂, NHC(NH)NH₂, ou NR₄R₅, dans lequel chacun des substituants R₄ et R₅ représente un atome H ou un groupement alkyle en C₁-C₅ qui est non-substitué ou substitué par un groupement hydroxyle ou amino ; ou un sel acide ou un N-oxyde de celui-ci qui est physiologiquement tolérable ; dans la production d'un médicament utile dans la prévention de la multidrogue résistance observée pendant le traitement des tumeurs qui est un schéma posologique à doses fractionnées ; ce traitement comporte une première administration et une seconde administration dudit compose, la seconde administration commençant entre 15 minutes et 24 heures après le début de la première administration.

8. Utilisation conformément à la revendication 7 dans laquelle le schéma posologique à doses fractionnées comprend au moins 2 administrations du médicament sur un intervalle allant de 30 minutes à 8 heures.

9. Utilisation conformément à la revendication 7 ou 8 dans laquelle le schéma posologique à doses fractionnées comprend au moins 2 administrations du médicament sur un intervalle allant de 30 minutes à 6 heures.

10. Utilisation conformément à l'une quelconque des revendications 7 à 9 dans laquelle le schéma posologique à doses fractionnées comprend au moins 2 administrations du médicament sur un intervalle allant de 30 minutes à 4 heures.

11. Utilisation conformément à l'une quelconque des revendications 7 à 10 dans laquelle le schéma posologique à doses fractionnées comprend entre 2 et 4 administrations du médicament sur un intervalle allant de 2 heures à 4 heures.

12. Utilisation conformément à l'une quelconque des revendications 6 à 11, dans laquelle la multidrogue résistance est exprimée par deux ou plus mécanismes de multidrogue résistance.

13. Utilisation conformément à l'une quelconque des revendications précédentes dans laquelle le schéma posologique à doses fractionnées comprend une troisième administration et des administrations supplémentaires dudit composé, la troisième administration et chaque administration supplémentaire commençant entre 15 minutes et 24 heures après le début de l'administration précédente.

14. Utilisation conformément à l'une quelconque des revendications 1 à 13 dans laquelle l'administration inclut l'administration d'un second composé qui inhibe les tumeurs et qui n'est pas un inhibiteur de la topo-isomérase II.

15. Utilisation conformément à la revendication 14, dans laquelle les deux composés sont administrés séparément ou en alternance.

16. Utilisation conformément à la revendication 14 ou à la revendication 15, dans laquelle la (les) doses(s)ou au moins une des doses constituantes de l'acridine carboxamide ayant la formule générale I' ou un sel ou un N-oxyde de celui-ci et le second composé qui inhibe les tumeurs sont administrés dans les deux jours l'un de l'autre.

17. Utilisation conformément à l'une quelconque des revendications 14 à 16, dans laquelle le second composé qui inhibe les tumeurs est la cisplatine, la cyclophosphamide, la bléomycine, le carboplatine, le décarbazine, la miomycine C, le melphalan, le fluorouracile, le 5-fluorodéoxyuridine, le méthotrexate, le taxol, la vincristine, la vinblastie, la vindésine, le tamoxifène, le facteur de nécrose tissulaire, l'interleukine II ou l'interféron.

18. Utilisation conformément à l'une quelconque des revendications 1 à 17, dans laquelle l'administration est faite chez un patient qui est atteint d'un sarcome, un mélanome, ou un cancer du poumon, du sein, des ovaires, des testicules ou du colon, ou des tumeurs cérébrales.

19. Utilisation conformément à la revendication 18, dans laquelle l'administration est laite chez un patient qui est atteint d'un mélanome, un cancer du colon avancé ou une tumeur cérébrale.

20. Utilisation conformément à l'une quelconque des revendications 1 à 19, dans laquelle au moins une des doses constituantes de l'acridine carboxamide ayant la formule générale I' ou un sel ou un N-oxyde de celui-ci est utilisé avec un autre composé qui est un agent lié à l'ADN qui réduit la toxicité de l'hôte du premier composé, cet autre composé et l'acridine carboxamide ayant la formule I' ou un sel ou N-oxyde de celui-ci étant présents dans la même formulation ou dans des formulations différentes et étant administrés simultanément ou séquentiellement.

21. Utilisation conformément à l'une quelconque des revendications précédentes dans laquelle le composé qui inhibe les tumeurs est le N-[2-(diméthyl-amino)éthyle]acridine-4-carboxamide.
